# EUROPEAN PATENT SPECIFICATION

(11) **EP 2 967 933 B1**
(45) Date of publication and mention of the grant of the patent: **23.05.2018**
(21) Application number: 14744147.1
(22) Date of filing: 14.03.2014
(51) Int. Cl.: A61F 2/88, A61F 2/91

(54) **HELICAL HYBRID STENT**
SPIRALFÖRMIGER HYBRIDSTENT
ENDOPROTHÈSE HYBRIDE HÉLICOÏDALE

(30) Priority: 14.03.2013 US 201313829153
(43) Date of publication of application: 20.01.2016
(60) Divisional application: 18155804.0
(73) Proprietor: Medinol Ltd., 6158101 Tel Aviv (IL)
(72) Inventor: RICHTER, Jacob, 4692000 Arsuf (IL); WEIZMAN, Oleg, 4636431 Herzliya (IL); BELOBROVY, Igor, 4922351 Petah-Tiqwa (IL)
(74) Representative: Kuhnen & Wacker Patent- und Rechtsanwaltsbüro PartG mbB
(86) International application number: PCT/IB2014/001121
(87) International publication number: WO 2014/140892

(56) References cited:
- EP-A1- 2 526 905
- EP-A1- 2 529 706
- US-A1- 2009 234 433

## Description

### FIELD OF THE INVENTION

The invention relates generally to stents, which are intraluminal endoprosthesis devices implanted into vessels within the body, such as blood vessels, to support and hold open the vessels, or to secure and support other endoprostheses in vessels.

### BACKGROUND OF THE INVENTION

Various stents are known in the art. Typically, stents are generally tubular in shape, and are expandable from a relatively small, unexpanded diameter to a larger, expanded diameter. For implantation, the stent is typically mounted on the end of a catheter with the stent being held on the catheter in its relatively small, unexpanded diameter. Using a catheter, the unexpanded stent is directed through the lumen to the intended implantation site. Once the stent is at the intended implantation site, it is expanded, typically either by an internal force, for example by inflating a balloon on the inside of the stent, or by allowing the stent to self-expand, for example by removing a sleeve from around a self-expanding stent, allowing the stent to expand outwardly. Some self-expanding stents are further explanded to their final diameters by a balloon. In all these cases, the expanded stent resists the tendency of the vessel to narrow, thereby maintaining the vessel's patency.

Stents may be constructed from tubes or from a flat sheet of metal, also interchangeably refered to herein as planar sheet of metal, which is rolled and fixed such as by welding, mechanical lock or otherwise, to form the tubular structure of the stent.

Some examples of patents relating to stent designs include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

US 2009/0234433 A1 discloses an expandable helical stent formed of an amorphous metal alloy or other non-amorphous metal with a securement. The stent is formed from flat or tubular metal in a helical coiled structure which has an undulating pattern. The main stent component may be formed of a single helically coiled component. Alternatively, a plurality of helically coiled ribbons may be used to form a stent heterogeneous in design, material, or other characteristic particular to that stent. The helical tubular structure may be secured with a securement, such as a weld, interlock or a polymer, to maintain the helical coils in a tubular configuration.

One type of stent is known as the helical or coiled stent. Such a stent design is described in, for example, U.S. Patent nos. 6,503,270 and 6,355,059. This stent design is configured as a helical stent in which the coil is formed from a wound strip of cells wherein the cells form a serpentine pattern comprising a series of bends. Other similar helically coiled stent structures are known in the art.

One object of prior stent designs has been to insure that the stent has sufficient radial strength when it is expanded so that it can sufficiently support the lumen. Stents with high radial strength, however, tend also to have a higher longitudinal rigidity than the vessel in which it is implanted. When the stent has a higher longitudinal rigidity than the vessel in which it is implanted, increased trauma to the vessel may occur at the ends of the stent, due to stress concentrations on account of the mismatch in compliance between the stented and un-stented sections of the vessel, or otherwise, the rigid stent may interfere with the vessel's natural tendency to bend and to stretch. Conversely, stents with good flexibility often lack sufficient and/or uniform radial support for the vessel wall. Thus, a continued need exists in the art for a stent having a balance of good radial strength and a high degree of longitudinal flexibility.

Another problem in the art arises when trying to simplify the manufacturing process of a stent to reduce costs yet prevent manufacturing defects, while still producing a stent with uniformly high flexibility and sufficient radial support.

### SUMMARY OF THE INVENTION

Insofar as the terms "invention" and/or "embodiment" are used in the following, and/or features are presented as being optional, this should be interpreted in such a way that the only protection sought is that of the invention as claimed. The present invention provides a helical stent that is longitudinally flexible such that it can easily be tracked down tortuous lumens and does not significantly change the compliance of the vessel after deployment, wherein the stent is relatively stable so that it avoids bending or tilting in a manner that would potentially obstruct the lumen and avoids leaving significant portions of the vessel wall unsupported. The stent of the present invention comprises a helical structure maintained by a polymer fiber layer or other securement. Further, this stent has the radial support of a metal stent combined with longitudinal flexibility, conformability and fatigue resistance to longitudinal repeated bending, compression and twisting, that is much higher than that achievable by metal stents.

One embodiment of the invention comprises a main stent component combined with a polymer fiber layer such as, for example, a biocompatible material, wherein the polymer fiber layer maintains the tubular shape of the stent while the main component provides structural support both to the vessel and the polymer fiber layer to prevent sagging of the polymer layer into the lumen upon deployment.

The main stent component may be formed of a ribbon or strip as a continuous elongated component, preferably having spaced undulating portions forming periodic loop portions. The undulating portions are understood to include portions having a generally sinusoidal or zig-zag pattern. The ribbon may be helically wound to produce a helical, tubular structure which can function to hold open a blood vessel upon expansion. The ribbon is designed so as to naturally form a helical, tubular structure upon helical winding such that the individual cycles of the helical coils -- defined by the length of the ribbon required to traverse the entire circumference of the resulting tubular structure in the helical direction -- are spaced apart from one another across the longitudinal axis of the tubular structure. The stent can also comprise two or more simultaneously wound ribbons, such that the windings of the different ribbons will interchange or alternate along the stent or will be partially or completely overlapped.

Alternatively, the main stent component or helically oriented ribbon may be formed from a tube wherein the tubular structure has been etched or laser cut into the helically coiled structure of the instant invention.

The main stent component forms a tubular structure of helical coils. The distance along the longitudinal axis of the stent between cycles of the helical coils may vary in length depending on the needs of the particular stent.

In another embodiment, the main stent component may be designed such that each undulating coil directly abuts an adjacent undulating coil of the helical structure so that the space between cycles is minimized; that is, the undulating pattern is nestled into an adjacent, substantially similar undulating pattern at different cycles of the helical coils. In this manner, the helical coils of the stent provide enhanced coverage of the wall of the lumen without loss of overall stent flexibility. Because the helical coils may be nestled into one another without directly touching each other, the overall flexibility of the formed stent is unaffected by the proximity of different cycles of the helical coils. This arrangement also prevents potential sagging of the polymer layer connecting the helix. The nestling of elements in adjacent coils can be either by nestling of undulating structures as described above or by nestling of any type of connected elements, connected to the undulating structure. These elements can be straight - stick like - elements aligned with the longitudinal direction of the stent or slanted or curved relative to it.

The main stent component may comprise side bands and end bands. The side bands extend in a parallel fashion along the length of the main stent component. Each preferably comprises an undulating pattern which may intersect directly with one or more adjacent side bands or through cross-struts. End bands may extend from either end of the strip and may be positioned at an angle to the side bands which form the central portion of the ribbon. These end bands may be designed to form a circumferential band or ring around the circumference of the tubular structure at either or both ends of the stent upon formation. The end bands may be tapered and/or affixed with additional elements, such as hooks, polymers, welds or the like to secure the ends of the helical tubular structure. Alternatively, the end bands may be formed by extending the length of a side band such that a single undulating pattern extends in either longitudinal direction of the main stent component. The main stent component may further comprise of one or more hooks extending from either or both side bands at an angle oriented to align with an end band upon formation of a tubular stent. Upon formation of the stent - such as, by example, helically winding the main stent component - the hook may be connected to the end band - either by welding or other means - to form a closed band or ring around the circumference of the stent; the band or ring may be oriented approximately at the barrel of a right cylinder having the longitudinal axis of the stent.

The main stent component may be formed from amorphous metal alloys, regular metals, or other biocompatible materials. Amorphous metal stents of the invention may be formed of one or more flat sheets of helically wound metal. Because amorphous metal alloys cannot be easily welded without the metal reverting to an undesirable crystalline form, the present invention contemplates wrapping or embedding the helically coiled amorphous metal alloy main stent component in a polymer fiber layer, such as a biocompatible non-metallic material, thereby forming a hybrid stent, where hybrid is taken to mean that the mechanical properties of the stent are a hybrid of a strong radial structure typical for metal and soft, flexible and durable longitudinal structure typical of non-metallic materials.

In one embodiment, the main stent component may be held in its helical coiled form by a polymer layer without requiring welding or otherwise interlocking the helically wound strip to itself. A second stent component, *i.e.,* a securement, may be used to provide longitudinal rigidity and structural support for the tubular shape of the main stent component while aiding in longitudinal flexibility of the stent. The securement is oriented and affixed to the main stent component such that, upon expansion or bending of the stent, the securement contributes to the overall flexibility of the stent while still aiding in maintaining the main stent component in a tubular shape. The securement may comprise fibers, wires, threads, ribbons, strips, polymers, meshes or the like. In another embodiment, the main stent component is held in its helical form by welding or interlocking elements of the helical coils to hold the structure in proper cylindrical shape. Similarly, embodiments are contemplated that would combine polymer and other securement means to maintain the helical structure.

### BRIEF DESCRIPTION OF THE DRAWINGS

Figure 1 illustrates a photomicrograph of stent members connected by a porous polymeric fiber structure.
Figure 2 illustrates stent having a schematic helical component connected by a fiber polymeric structure.
Figure 3 illustrates one embodiment of a main stent component connected by a fiber polymeric structure.
Figure 4 illustrates a flat or planar ribbon main stent component formed according to one embodiment of the invention.
Figure 5 illustrates a helical main stent component according to the invention having variable distances between helical coils.
Figure 6 illustrates another embodiment of the invention having a helical main stent component having side bands and end bands, detailing varying cross struts, and embedded in a polymer.
Figure 7 illustrates yet another embodiment of the invention wherein the helical main stent component has its coils nestled into one another.
Figure 8 illustrates an embodiment of a main stent component composed of a flat ribbon having a patterned band and comprises struts with one or more exemplary fenestrations.
Figure 8A is an enlarged view of an end band of the main stent component of Figure 8.
Figure 9 illustrates a flat ribbon view of a main stent component having undulations and comprising struts with one or more exemplary fenestrations.
Figure 9A is an enlarged flat ribbon view of a first end band of Figure 9.
Figure 9B is an enlarged flat ribbon view of a second end band of Figure 9.
Figure 10 illustrates a photograph of a securement structure and a main stent component.
Figure 11 illustrates an embodiment of the helical main stent component embedded in several ribbon securements.
Figure 12 illustrates a helical main stent component maintained by a plurality of helical securements fastened at discrete points.
Figure 13 illustrates a flat or planar ribbon view of a main stent component having side bands with undulations with end bands having undulations extending from either end of the side bands, as well as hooks extending from each of the side bands.
Figure 14 illustrates a tubular view of the helical main stent component of Figure 13.
Figure 14A is an enlarged partial view of the stent of Figure 14.

### DETAILED DESCRIPTION OF THE INVENTION

This invention provides a new class of intraluminal prosthetic devices defined as helical hybrid stents. In particular, the stents of the present invention comprise a main stent component in the form of a helical tubular structure. The main stent component may be held in its coiled position by a second component, securing the helical coils into a tubular structure. The second component may be one or more of a variety of means for securing the main stent component in the tubular form. The second component may be, for example, weld points, interlocking means and/or a polymer. In one embodiment, the second component comprises a polymer or polymer fibers which wraps around or embeds itself in the coiled main stent component. The elastic range of the polymer fiber layer must be sufficient to allow expansion of the stent and maximal bending during and after implantation without reaching the elastic limit.

The stent of the present invention may be balloon expandable or self-expanding, or first self-expanding and then further expanding by a balloon. When a balloon-expandable stent system is used to deliver the stent, the stent is mounted on the balloon and the catheter assembly is positioned at the implantation site. The balloon is then inflated, radially applying a force inside the stent and the stent is expanded to its expanded diameter. Alternatively, the stent may be self-expanding in which case a balloon may not be needed to facilitate expansion and delivery of the stent.

By forming a stent with a single main stent component instead of separate components, the present invention provides for ease of manufacturing a whole stent structure without the necessity of forming multiple components and thereafter joining them to form a stent. The present invention also allows for the manufacturing of a stent formed of two or more simultaneously coiled main stent components which may or may not be of the same material or design, such that the windings of different ribbons may interchange, or alternate over the length of the stent. The present invention also allows for forming a stent from hard-to-weld materials, such as amorphous metal without the need to fix the individual rings.

The present invention relates to a stent comprising a continuous main stent component having side bands containing a periodic series of undulations that is helically arranged, for example, as a coil into a helical, tubular shape. The main stent component may be formed from one or more flat metal ribbons. Alternately, the main stent component may be formed as a tube wherein a helically coiled pattern has been etched or laser cut into it. In either case, the helical stent will have a pattern resembling a coiled ribbon or ribbons, wherein each ribbon comprises two or more parallel side bands each having an undulating pattern. The side bands are joined together directly and/or through cross-struts.

The main stent component may further comprise end bands, which have undulating bands that may extend at an angle from each end of the main stent component with the end bands extending in the general direction of the side bands. The end bands in this orientation each follow the circumferential axis of the helically coiled tubular structure. Alternatively, the end bands may extend in a direction generally parallel with the side bands of the main stent component, oriented to align, upon helical formation of the stent, with hooks extending at an angle from the main stent component. Optionally, the side bands of the ribbon may be tapered without resort to additional end bands. Both the end bands and tapering of the ends of the main stent component allow the ends of the finished stent to be substantially straight; *i.e.,* it allows the stent to form a right cylinder, with each of the ends of the cylindrical stent lying in a plane perpendicular to the longitudinal axis of the stent.

The cross-struts may be straight connectors or may have one or more loops between connection points to side bands and/or end bands. Further, individual cross-struts may connect an end band to an adjacent side band while other cross struts connect two adjacent end bands one to another or two adjacent side bands one to another.

The undulating patterns of the side bands and end bands are such that, in the helically coiled form of the ribbon, the adjacent side bands and/or end bands may be substantially parallel to one another. The undulating patterns are understood to have peaks and troughs. The troughs may be defined by points of connection to the cross-struts or to troughs of the adjacent-most side band or end band. The end bands are arranged at an angle such that the end bands extend about a circumferential axis of the helically coiled main stent component.

The end sections may be formed from the same ribbon which constitutes the side bands. The end sections support the helical coiled structure. Alternatively, the helical coils of the main stent component may be connected by separate end band elements aligned with the longitudinal direction of the stent or slanted relative to it.

The ribbon may be arranged to provide a cellular stent design. The helical main stent component can be any structure which provides a stored length to allow radial expansion. Examples of such specific designs are described in, but not limited to, U.S. Patent No. 6,723,119. Another example design is a stent pattern described in U.S. Patent No. 7,141,062 (" '062"). The '062 stent comprises triangular cells, by which is meant a cell formed of three sections, each having a loop portion, and three associated points of their joining forming each cell. One or more rows of such cells may be assembled in a ribbon which may be helically coiled from two or more side bands to form a main stent component. Similarly, the cells in the stent described in U.S. Patent No. 5,733,303 to Israel et al. (" '303") may be used for the main stent component but helically coiled. The '303 patent describes a stent having cells formed of four sections, each having a loop portion and four associated points of their joining forming each cell, also known as square cells. Such square cells may be formed with the side bands and cross struts of the helically coiled ribbon of the present invention. Each of these designs is expressly incorporated herein *in toto* by reference. Other similarly adaptable cellular stent designs known in the art are readily applicable to the helical stent of the present invention.

Employment of a light and porous or fiber polymeric material in the stents of the present invention provides several advantages. For example, a fibrous material may provide a longitudinal structure thereby enhancing the overall flexibility of the stent device. Such a material may be applied to a tubular stent in a continuous or non-continuous manner depending upon the particular needs of the structure contemplated. Polymeric material can form a porous fiber mesh that is a durable polymer. The longitudinal polymeric structure serves at least two functions. First, the longitudinal polymeric structure is more longitudinally flexible than a conventional metallic structure. Second, the polymeric material is a continuous structure with small inter-fiber distance and can be used as a matrix for eluting drug that would provide a more uniform elution bed. Another advantage of using these materials is that the continuous covering provided by the material after the stent is deployed in a vessel is believed to inhibit or decrease the risk of embolization. Yet another advantage is the prevention of "stent jail" phenomenon, or the complication of tracking into side branches covered by the stent. Further advantage is the high fatigue resistance of polymer structures with high elastic range.

The polymer layer can be disposed within interstices and/or embedded throughout the stent. The polymer layer may secure portions of the stent structure or may fully envelop the entire stent. The polymer layer is a biocompatible material. Biocompatible material may be a durable polymer, such as polyesters, polyanhydrides, polyethylenes, polyorthoesters, polyphosphazenes, polyurethane, polycarbonate urethane, silicones, polyolefins, polyamides, polycaprolactams, polyimides, polyvinyl alcohols, acrylic polymers and copolymers, polyethers, celluiosics and any of their combinations in blends or as copolymers. Of particular use may be silicone backbone-modified polycarbonate urethane and/or expanded polytetrafluoroethylene (ePTFE).

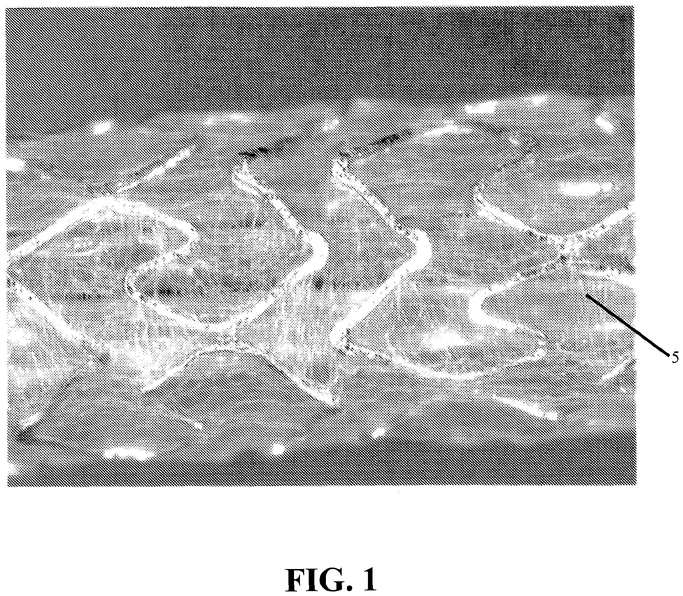

FIG. 1 shows a photomicrograph of an exemplary stent illustrating stent members connected by a porous polymer layer. The stent of Figure 1 is connected by a polymer layer 5 represented here as a porous longitudinal structure along a longitudinal axis of the stent. Illustrated here, the polymer layer 5 is a porous durable fiber mesh. The polymer layer 5 provides a continuous structure having small inter-fiber distances and forming a matrix. This matrix may be used for eluting a drug and may provide a uniform elution bed over conventional methods. In addition, the polymer layer 5 may function to hold the main stent component in a tubular shape and to prevent unwinding upon expansion and flexing. In addition, the polymer layer 5 enables longitudinal flexibility to the stent structure.

The longitudinal structure of the biocompatible polymer layer may be porous or it may be formed as a tube with fenestrations or a series of fibers with spaces between them, to promote growth of neo-intima that will cover the stent and secure it in position. Fenestrations may also promote better stabilization of the stent. The shape of fenestration can be made in any desired size, shape or quantity.

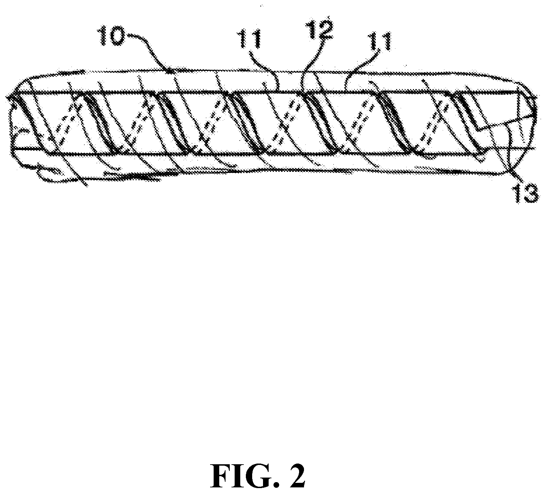

FIG. 2 shows an example helically coiled ribbon 12 disposed in a polymer layer such as a porous fiber mesh 10. As shown in FIG. 2, the stent is formed as a helically wound ribbon having ends 13 and coils 11. Depending on the embodiment, the coils 11 of the ribbon 12 are relatively resistant to longitudinal displacement or tilting because of the width of the ribbon 12. The mesh 10, although allowing longitudinal flexibility of the stent, further provides support to the stent to resist longitudinal displacement or tilting. The ribbon 12 is designed to have a helical tubular shape.

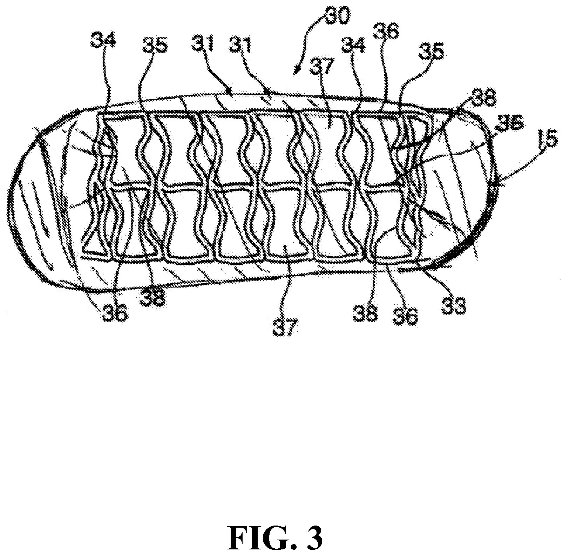

FIG. 3 shows a serpentine coiled ladder stent 30 constructed in accordance with the invention. The serpentine coiled ladder stent 30 in FIG. 3 is shown having a porous fiber mesh 15 disposed about the stent.

The serpentine coiled ladder stent 30 embodiment illustrated in FIG. 3 is configured as a helical stent in which the coils are formed from a helical strip of cells 37, wherein the sides of the cells 37 are serpentine or contain undulations. The stent in this illustration is comprised of a strip helically wound into a series of helical coils 31, wherein the main stent component is formed of two side bands 34, 35 connected to each other, for example by a series of cross struts 36. Each side band 34, 35 is formed in a serpentine pattern comprising a series of undulations 38. Upon expansion of the stent, the undulations 38 of the side bands 34, 35 open to increase the length of each of the individual cells 37 in the helical direction. Thus, lengthening the strip in the helical direction permits the stent 30 to expand without any significant unwinding of the strip, or foreshortening. In the unexpanded state, the side bands collapse to form a serpentine continuum.

In the illustrated embodiment of FIG. 3, the cross struts 36 joining the side bands 34, 35 to each other are straight and extend in a direction generally perpendicular to the helical direction in which the strip is wound. Alternatively, the cross struts may have one or more bends, and/or they may extend between the two side bands at other angles. In the illustrated embodiment, the cross struts 36 join oppositely facing undulations 38 on the side bands 34, 35, and they are attached to the side bands 34, 35 at every second undulation 38. Alternatively, the cross struts 36 may be joined in other places, and may occur with more or less frequency, without departing from the general concept of the invention. The side bands 34, 35 and the cross struts 36 form the perimeter of each cell. The stent alternatively may be formed without cross struts 36, by having, for example, the two serpentine side bands 34, 35 periodically joined directly to each other at adjacent points.

Furthermore, as shown in FIG. 3, the ends 33 of the serpentine main stent component may be tapered. The tapering of the ends 33 of the main stent component allows the ends of finished stent to be straight, *i.e.,* it allows the stent to take the form of a right cylinder, with each of the ends of the cylindrical stent lying in a plane perpendicular to the longitudinal axis of the stent. The ends 33 of the main stent component may be joined to respective adjacent windings 31 using the porous fiber mesh 15 to join ends 33, for example when made from an amorphous metal.

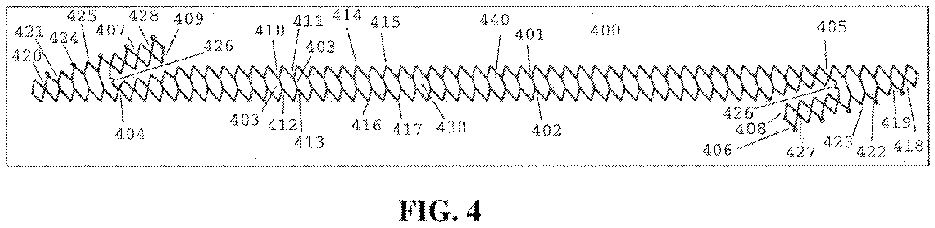

FIG. 4 illustrates an embodiment of the invention wherein the main stent component is shown in the planar ribbon form. The main stent component 400 is shown in an uncoiled state, *i.e.,* planar or flat. As depicted in FIG. 4, the main stent component 400 has an undulating design in the longitudinal direction. The undulating design comprises a first side band 401 having an undulating shape and a second side band 402 having an undulating shape. The first side band 401 and second side band 402 are arranged along a generally parallel orientation except at either end of the side bands where the first side band tapers toward the second side band and the second side band tapers toward the first side band. Accordingly, when the main stent component 400 is laid flat as depicted in FIG. 4, the undulations of the first side band 401 comprise troughs (*e.g.,* 410, 411) that face toward the second side band 402 and peaks (*e.g.,* 414, 415) that face away from the second side band 402. Similarly, the undulations of the second side band 402 comprise troughs (*e.g.,* 412, 413) that face toward the first side band 401 and peaks (*e.g.,* 416, 417) that face away from the first side band 401. The first side band 401 and second side band 402 are connected to each other by a plurality of first cross struts 403 to form cells 440. In particular, for example, at least one trough (*e.g.,* 410) of the first side band 401 is connected to a corresponding trough (*e.g.,* 412) of the second side band 402 via a first cross strut 403. Thus, a series of cells are formed, each cell defined individually by the joining of the adjacent side bands to form an enclosed space by cross-struts. For example, in FIG. 4, a cell is defined by the portion of the first side band between troughs 410 and 411, the portion of the second side band between troughs 412 and 413 and the first cross-struts 403 respectively connecting troughs 410 and 412 and inner peaks 411 and 413.

In FIG. 4, the first cross struts 403 connect first side band 401 and second side band 402 at regular intervals, in particular at adjacent troughs, thereby forming cells, *e.g.,* 430. In alternative embodiments, the number of first cross struts 403 may differ from that illustrated in FIG. 4. For example, the first cross-struts 403 may connect the first band 401 and second band 402 at regular intervals at, for example, every second trough, or every third trough, or every fourth trough, etc., thereby making larger cells. In still other embodiments, the first cross struts 403 may connect the first side band 401 and second side band 402 at varying intervals, for example, the varying interval pattern may be: adjacent trough, third trough, adjacent trough, fourth trough, adjacent trough, third trough, etc. (not shown), or another pattern, as may be appropriate for a particular use, thereby making a variety of differently sized cells along the main stent component. The first cross-struts 403 may each have the same width relative to each other and to the side bands 401, 402, as shown in FIG. 4. Alternatively, the first cross-struts 403 may have a different width from the first and second side bands 401, 402, or a different width from each other, as appropriate for a particular use. In addition, first cross-struts 403 may comprise a straight member or may contain one or more loops, thereby forming square cells similar to those taught by the '303 patent or triangular cells as taught in the '062 patent. The cross struts may connect adjacent or offset troughs of the first and second side bands 401, 402. As shown in FIG. 4, differently shaped cross-struts, or no cross-struts may alternatively be employed in a single stent design depending on the particular use of the stent so that a stent having different cell shapes may be formed.

The main stent component 400 in the embodiment depicted in FIG. 4 tapers at each end. In particular, the length of the cross struts 403 shorten toward each end of the main stent component 400, so that the first and second bands 401, 402 become closer together and eventually are connected directly at points of connection 404 and 405. Alternatively, in embodiments without cross struts, the undulations may become more shallow to create a tapered end on the flattened ribbon of the main stent component.

Extending from the end of either side band 401 and 402 in FIG 4 are end bands 406 and 407. Thus, a first end band 406 extends from the end of the first side band 401 in a direction offset from the general direction of the first side band 401. A second end band 407 extends from the end of the second side band 402 in a general direction offset from the general direction of the second side band 402 and opposite the first end band. The first end band 406 and second end band 407 each have an undulating pattern. The first end band 406 has troughs (*e.g*. 418, 419) that face toward the first side band 401 and peaks (*e.g.* 422, 423) that face away from the first side band 401. Likewise, the second end band 407 has troughs (*e.g.* 420, 421) that face toward the second side band and peaks (*e.g.* 424, 425) that face away from the second side band 402. The first end band 406 connects directly to the first side band 401 at, *e.g.,* trough 418; however, as the first end band 406 angularly extends away from the first side band, second cross-struts 426 connect the first end band 406 to the first side band 401. Likewise, the second end band 407 connects directly to the second side band 402 at, *e.g.,* trough 420; however, as the second end band 407 angularly extends away from the second side band, second cross-struts 426 connect the second end band 407 to the second side band 402. As depicted in FIG. 4, the second cross struts 426 may contain one or more loops between points of connection with adjacent end bands and/or side bands. The peaks of the first end band 406 and second end band 407 optionally may have additional circular structures extending from the peaks (*e.g.* 423, 424) as depicted by FIG. 4.

In addition, a third end band 408 is arranged generally parallel to first end band 406, with troughs facing each other and connecting directly, *e.g.* 427, to said first end band. A fourth end band 409 is arranged generally parallel to second end band 407, with troughs facing each other and connecting directly, *e.g.* 428, to said second end band. The third end band 408 and fourth end band 409 each have an undulating pattern.

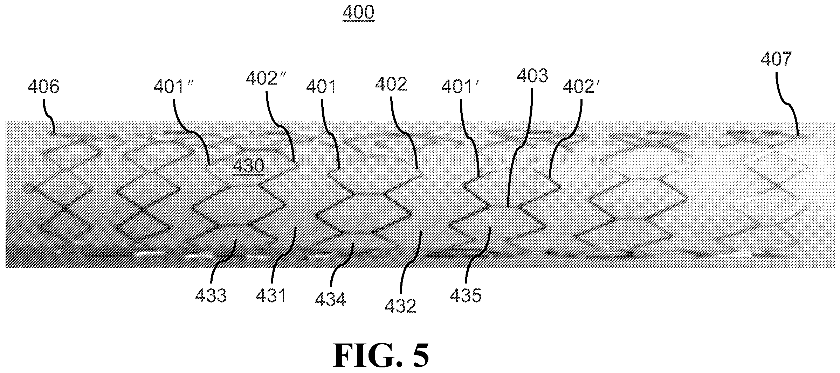

FIG. 5 illustrates a helically coiled stent wherein the main stent component 400 forms a tubular structure and the end bands of the ribbon secure the ends of the tubular structure. The undulating design of the main stent component 400 forms a helical, tubular structure, in which the coils of the helix self-arrange to create variable and/or uniform spacing along the longitudinal axis of the tubular structure, *e.g.* 431, 432, between helical cycles, *e.g.* 433, 434, 435, as depicted in FIG. 5. Because the stent 400 forms a helix, the first side band 401 and the second side band 402 of the ribbon, may be spaced apart to various extents.

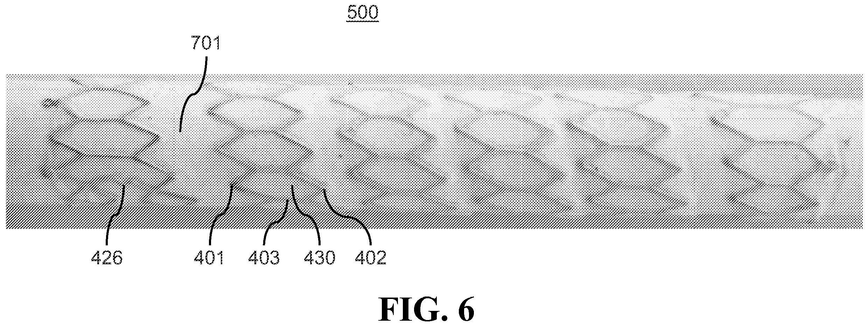

The helical main stent component 500 may also be secured by embedding the tubular structure in a longitudinal polymer layer as in FIG. 5 and/or FIG. 6, rather than by locking mechanisms or welding alone. The longitudinal polymer layer comprises a biocompatible material. The stent in FIG. 6 is rotated slightly compared to that in FIG. 5 so that the second cross-strut 426 having a loop is visible. Also identified are the first band 401, the second band 402, a first cross strut 403, and a cell 430.

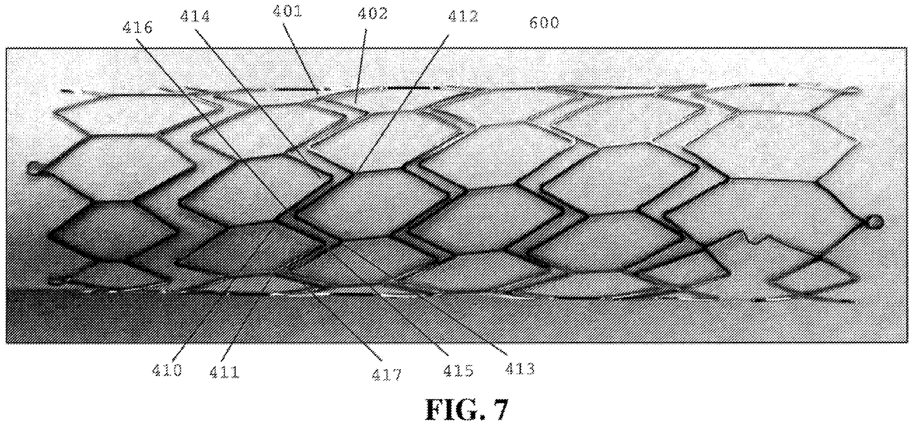

FIG. 7 illustrates a stent according to the invention wherein the helical coils are positioned so that little or no substantial longitudinal space exists between cycles of the helical coils. That is, as illustrated by FIG. 7, the peaks (*e.g.* 414, 415) of the first side band 401 nestle into the circumferential area created by the peaks (*e.g.* 416, 417) of the second side band such that the peaks 414, 415 of the first side band 401 approach the troughs 412, 413 of the second side band 402; yet, the first side band 401 remains substantially parallel to the second side band 402. Likewise, the peaks (*e.g.* 416, 417) of the second side band 402 nestle into the circumferential area created by the peaks (*e.g.* 414, 415) such that the peaks 416, 417 of the second side band 402 are in close proximity to the troughs 410, 411 of the first side band 401. It may be desirable to position the nestled side bands so that no direct contact occurs between first side band 401 and second side band 402. Because the first side band 401 and the second side band 402 have substantially similar design, the first side band 401 and the second side band 402 can approach one another in this fashion over the entire length of the formed stent. In this manner, the first side band 401 and the second side band 402 may be described as nestled to one another. The stent of FIG. 7 has the additional advantage that the nestled pattern of adjacent first and second side bands minimizes the unsupported areas of the vessel wall and/or polymer layer to prevent sagging of the polymer layer into the lumen upon expansion without any loss of flexibility to the stent. In addition, the nestling of the helical coils separately facilitates the maintenance of the structure in the tubular form.

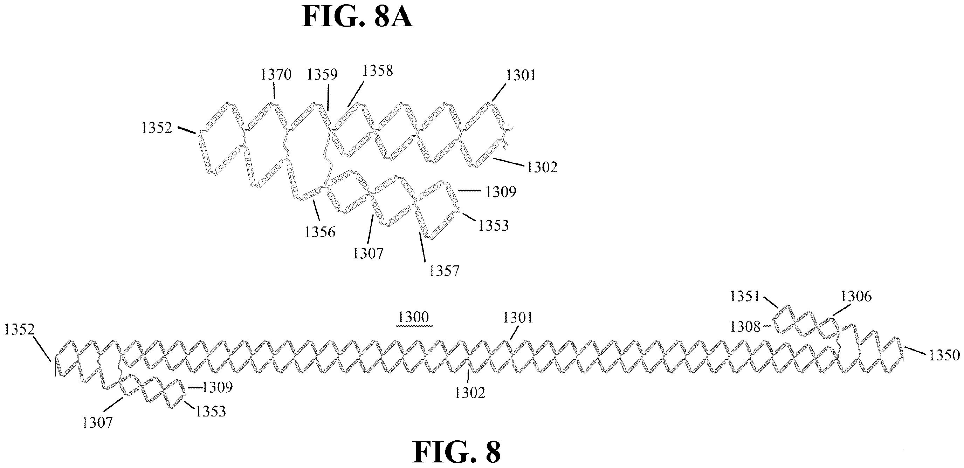

FIG. 8 illustrates an alternative embodiment wherein the main stent component 1300 is laid out in planar form, i.e., uncoiled. As depicted, the main stent component 1300 has a patterned band in the longitudinal direction. Like the embodiment of FIG. 4, the design of the main stent component 1300 in FIG. 8 contains a first side band 1301, a second side band 1302, a first end band 1306, a second end band 1307, a third end band 1308 and a fourth end band 1309. In the tubular form, side bands 1301 and 1302 form a continuous helical winding for the central portion of the stent body while first and second end bands 1306 and 1307 form right cylinders to the longitudinal axis of the stent for the end rings of the stent. In the first end band, first edge 1350 is brought together with second edge 1351 while, in the second end band, first edge 1352 is brought together with second edge 1353. Main stent component 1300 comprises struts having one or more fenestrations into which a therapeutic substance may be deposited.

Each band is formed with struts of sufficient width to include one or more fenestrations as shown, for example, in FIG. 8. The fenestrated struts of main stent component 1300 may be of any geometric shape, including, but not limited to, round, oval or rectangular. Further, the fenestrations may extend through the entire thickness of the strut (full fenestrations), or may extend only partially through (partial fenestrations), being open only on one side of the strut (luminal or abluminal in the tubular form). Also, the stent may have struts containing fenestrations having variable sizes, numbers and shapes on one strut or between different struts. The invention contemplates struts having full and/or partial fenestrations on either or both of the side and/or end bands. The struts defining the peaks and troughs of the side bands may vary in length along the length of the main stent component to accommodate the desired shape for the resulting helically coiled stent structure and the number of fenestrations. For example, in FIG. 8A, side band struts 1358 and 1359 differ in length as do end band struts 1356 and 1357. The fenestrated struts are connected by loops or turns 1370 wherein the material is narrower than that of the fenestrated struts to provide enhanced flexibility.

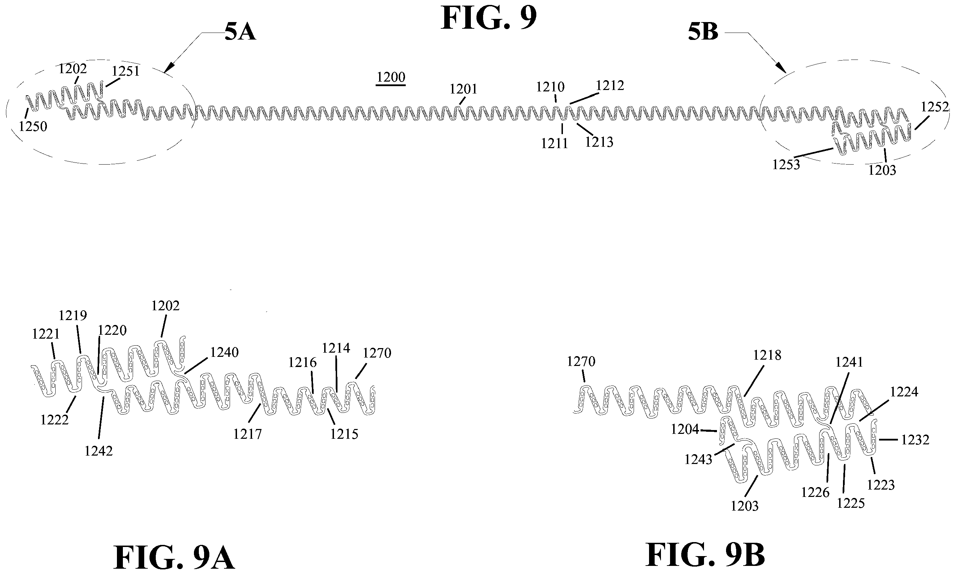

FIG. 9 illustrates yet another embodiment of the invention where the main stent component 1200 is laid out in flat form, i.e., uncoiled. As depicted, the main stent component 1200 is a single side band 1201 in the longitudinal direction when laid flat. Side band 1201 is attached to first end band 1202 and second end band 1203 by cross-struts 1240 and 1241, respectively. Side band 1201 comprises an alternating pattern of peaks (e.g., 1210, 1212) and troughs (e.g., 1211, 1213) defined by struts having the same or variable lengths. Each side and end band is formed with struts having sufficient width to include one or more full or partial fenestrations, as described above for FIG. 8, and are also applicable to FIG. 9. The fenestrated struts are connected by loops or turns 1270 that are narrower than that of the fenestrated struts to provide enhanced flexibility. As shown in FIG. 9A, the struts are of varying length and vary in the number of fenestrations in each strut. For example, strut 1217 has a different length and number of fenestrations than strut 1215. Strut 1216 has a different length but the same number of fenestrations than strut 1215. And struts 1214 and 1215 have the same lengths and number of fenestrations. The stent of FIG. 9A contemplates that struts (e.g., 1217) near the ends of the first side band 1201 may have different lengths than struts 1214 and 1215 and are configured to aid in helical winding.

End bands 1202 and 1203 form circumferential end rings in its tubular formd. The first end band 1202 and second end band 1203 extend from the ends of the side band 1201 in a direction angularly offset from the general direction of the side band 1201. End bands 1202 and 1203 are configured to form the ends of a right cylinder at the ends of the stent, flanking the helical windings of the central stent body upon winding of the structure into a stent. First end band 1202 has first edge 1250 and second edge 1251. In the tubular form, first edge 1250 is brought together with second edge 1251 to form a right cylinder to the longitudinal axis of the stent. Second end band 1203 has first edge 1252 and second edge 1253. In the tubular form, first edge 1252 is brought together with second edge 1253 to form a right cylinder to the longitudinal axis of the stent. As further explained below, the edges (1250 and 1251; 1252 and 1253) may be permanently affixed, or as an alternative, may be held in position with a securement which may keep the two edges in close proximity to maintain a right cylinder to the longitudinal axis of the stent.

In FIG. 9A, first end band 1202 comprises a set of undulations. The direction of the first end band 1202 is offset at an angle to the direction of the side band 1201. In FIG. 9A, the first end band extends from the side band is at an angle less than 45 degrees to the central body of the stent when the stent is planar. The undulating pattern of the first end band 1202 comprises alternating peaks (e.g., 1219, 1221) and troughs (e.g., 1220, 1222). Troughs (1220, 1222) of the first end band extend in the direction of the side band while the peaks (1219, 1221) point away from the side band. First end band 1202 also may contain struts having fenestrations. In FIG. 9A, cross-links 1240 and 1242, for example, connect the side band to the first end band. Cross-links 1240 and 1242 extend from the troughs of the first end band to the peak of the side band. Cross-links extending between the side band and the first end band are flexible connectors having one or more curved portions. The invention also contemplates an embodiment where the cross-links may contain one or more loops.

In FIG. 9B, second end band 1203 also comprises a set of undulations. The direction of the second end band 1203 is angularly offset to the direction of the side band
1201. Preferably, the second end band extends from the side band at an angle less than 45 degrees to the central body of the stent when the stent is laid flat. The undulating pattern of the second end band 1203 comprises alternating peaks (e.g., 1223, 1225) and troughs (e.g., 1224, 1226). Troughs (1224, 1226) of the second end band extend in the direction of the side band while the peaks (1223, 1225) point away from the side band. Second end band 1203 may contain struts having fenestrations. In FIG. 9B, cross-link 1241 connects the side band to the second end band. Cross-link 1241 extends from the trough of the second end band to the trough of the side band. Cross-links extending between the side band and second end band are flexible connectors having one or more curved portions. Cross-links connecting the side band to the second end band may comprise at least one loop.

In addition, the invention contemplates other end bands similar in construction to first and second end bands and connected to either the first or second end bands to facilitate helical winding and uniform coverage. In FIG. 9B, a third end band 1204 having fenestrated struts is connected to the second end band by cross-link 1243. As illustrated in FIGS 9, 9A and 9B, the invention contemplates first and second end bands which are not identically connected to the undulating or patterned side bands and which are not identical to each other. Like the side band, any one or all the end bands may comprise struts sufficiently wide to accommodate one or more full or partial fenestrations which are connected together with loops having a narrower gauge than the fenestrated struts.

The main stent component may be held in a helically wound position by a second component, securing the helical windings into a tubular structure. The second component, referred to herein as a securement, may be one or more of a variety of means for securing the main stent component in the tubular form. The second component may be, for example, weld points, interlocking means and/or a polymer. The securement maintains the helical winding of the central stent body and/or the formation of right cylinders by the end bands. In one embodiment, the securement comprises a structure in the form of fibers, sheets, threads or ribbons which are wrapped around or itself embedded in the coiled main stent component. In another embodiment, wires or ribbons formed of a metal or non-metal material maintain the main stent component in its tubular configuration. The securement comprises a material that allows flexibility and expansion of the helical main stent component without tearing or detachment of the securement and allows movement between the coiled windings of the main stent body relative to each other. Such a material may be applied to a tubular stent in a continuous or non continuous manner depending upon the particular needs of the structure contemplated.

Preferably, the securement allows expansion of the stent and maximal bending during and after implantation without reaching the elastic limit. The elastic range may be a product either of inherent elasticity in the material used, such as with certain polymers, or of the inclusion of a reserve length of a non-elastic material between points of connection with the main stent component. Yet another advantage of a securement is the prevention of "stent jail" phenomenon, or the complication of tracking into side branches covered by the stent. A further advantage is the high fatigue resistance of particular securement structures with high elastic range.

In one embodiment, the securement is a polymer that is a biocompatible material. Biocompatible material may be durable, such as polyesters, polyanhydrides, polyethylenes, polyorthoesters, polyphosphazenes, polyurethane, polycarbonate urethane, silicones, polyolefins, polyamides, polycaprolactams, polyimides, polyvinyl alcohols, acrylic polymers and copolymers, polyethers, celluiosics and any of their combinations in blends or as copolymers. Of particular use may be silicone backbone-modified polycarbonate urethane and/or expanded polytetrafluoroethylene (ePTFE). Any polymer having a high elastic ratio (high elongation factor within the elastic range) is particularly suitable for a securement. The polymer may also be porous. In embodiments where the polymer a continuous structure with small inter fiber distance, it may also be used as a matrix for eluting drug thereby providing a uniform elution bed. This type of porous securement may be applied to any other stent structure.

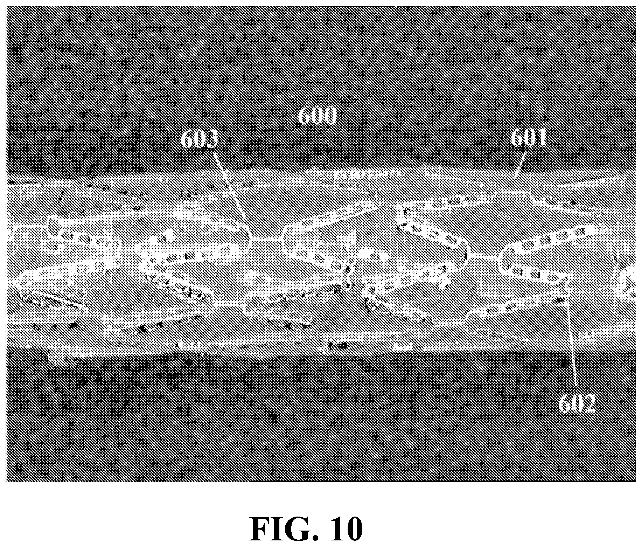

FIG. 10 shows the coiled main stent component 600 of FIG. 8, described above, wherein a porous and durable polymer securement 601 is applied over main stent component 600. Two adjacent struts of a first side band are connected to one another by turn 602, which includes a "dimple". The inclusion of a dimple in the turns is an optional feature depending upon the desired properties of the resulting stent. FIG. 10 also illustrates turn 603 which is without a dimple, and is employed in this embodiment at points where cross-struts connect the first side band to the second side band.

Polymeric securements as described above may also be employed in the form of threads, wires or ribbons, thereby securing the main stent component through, for example, a series of points of connection with the main stent component. One or more securement threads, wires or ribbons may be coiled around the stent in a helically different direction than the main stent component. In particular, the thread, wire or ribbon may be coiled around the stent in the reverse helical orientation from the direction of the helically wound strip. Alternatively, securements may be arranged along a longitudinal axis of the stent. Arranged in any non-parallel direction with the main stent component, each thread, wire or ribbon may overlap with the main stent component in a regular pattern across the length of the stent and may effectively function to secure the helical stent body structure. The securement thread, wire or ribbon may be affixed to the main stent component at one or more points of overlap through a variety of means, e.g., welding, bonding, embedding, braiding, weaving, crimping, tying, press fitting or the like, including also joining by adhesive means, e.g., gluing, dip coating, spray coating or the like. The polymeric securement may also be injected into a mold with or without the stent and hence become integrated within the stent. The threads, wires or ribbons maintain the tubular shape of the stent, while the longitudinally flexible quality of the polymeric material discussed above will enhance the overall flexibility of the stent.

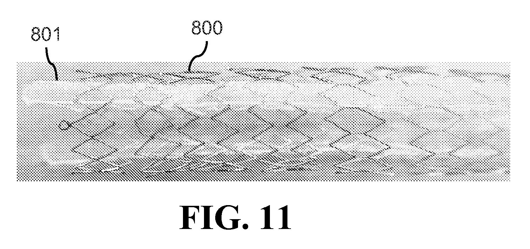

FIG. 11 illustrates a helically coiled stent wherein the main stent component 800 forms a helically oriented tubular structure that is secured in place by two ribbons 801. The ribbons 801 are a polymeric material that extend along the length of the stent. The ribbons may be affixed to the outside or the inside surface of the stent, or may be embedded in the helically coiled main stent component. In FIG. 11, the main stent component 800 is embedded within each ribbon 801 at points where the main stent component 800 and each second component ribbon 801 intersect.

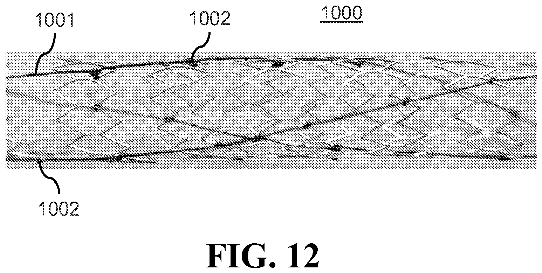

FIG. 12 illustrate a helically coiled stent wherein the main stent component 1000 forms a tubular structure similar to FIG. 5 and one or more securement wires 1001 are coiled in a different helical direction to that of the coiled central body portion of the stent. The securement wires 1001 are affixed to the main stent component 1000 at various points of connection 1002 along the stent, thereby maintaining the helical, tubular structure.

In addition to polymeric securements, any other suitable material, including metals and/or non-metals, may be employed as securements in the form of threads, wires or ribbons to secure the main stent component. The metal or non-metal securement wire, thread or ribbon may be affixed to the main stent component where they overlap through one or more of a variety of means as identified above. If the material employed to manufacture the second component is of a lesser longitudinal flexibility than desired, increased flexibility may be achieved by increasing the length of the thread, wire or ribbon between points of connection, thereby providing reserve length of the second component that can extend upon expansion or bending of the stent.

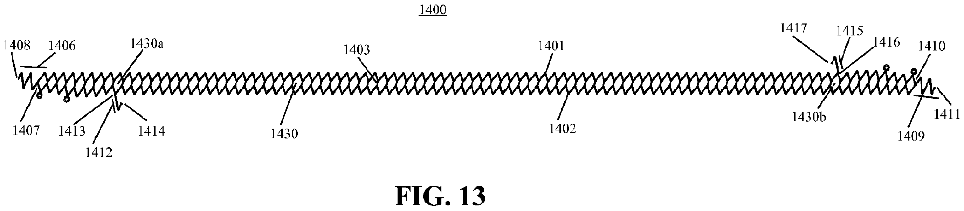

FIG. 13 illustrates an embodiment of the invention wherein the main stent component is shown, for illustration purpose only, in the flatten ribbon form. The main stent component 1400 has a cellular design, comprising a first side band 1401 having an undulating shape and a second side band 1402 having an undulating shape. The first side band 1401 and second side band 1402 are arranged in a generally parallel orientation except at either end of the side bands where,₌on one end₌the first side band tapers toward the second side band and on the other end the second side band tapers toward the first side band. The first side band 1401 and second side band 1402 are connected by struts 1403 to form cells 1430. Extending from the end of either side band 1401 and 1402 in FIG 13 are end bands 1406 and 1409. Thus, a first end band 1406, comprising a series of struts forming a first undulating pattern, extends from the end of the first side band 1401 in a direction generally parallel with the first side band 1401. The first end band 1406 has a first end 1407, located at the point at which the second side band 1402 tapers to connect with the first side band 1401, and a second end 1408 with a plurality of undulations therebetween. A second end band 1409, comprising a series of struts forming a second undulating pattern, extends from the end of the second side band 1402 in a direction generally parallel with the second side band 1402. The second end band 1409 has a first end 1410, located at the point at which the first side band 1401 tapers to connect with the second side band 1402, and a second end 1411 with a plurality of undulations therebetween. The first end band 1406 and second end band 1409 are each formed by an undulating pattern.

Extending from the main stent component is a first hook 1412 and a second hook 1415. The first hook 1412 extends directly from the second side band 1402 and has a first end 1413 that connects directly to a cell 1430a near one end of the main stent component 1400. The first hook 1412 further has a second end 1414. The second hook 1415 extends directly from the first side band 1401 and has a first end 1416 that connects directly to a cell 1430b near the opposite end of the main stent component 1400 from cell 1430a. The second hook further has a second end 1417. The first hook 1412 and the second hook 1415 extend in opposite directions from each other relative to the main stent component 1400. The first hook 1412 is positioned and oriented such that the second end 1414 of the first hook 1412 will align with the second end 1408 of the first end band 1406 in the tubular form of the stent. The second hook 1415 is positioned and oriented such that the second end 1417 of the second hook 1415 will align with the second end 1411 of the second end band 1409 in the tubular form of the stent.

The cells 1430 of the main stent component 1400 may be formed in a variety of sizes and shapes. FIG. 13 illustrates an embodiment of the invention wherein the main stent component 1400 has cells 1430 that are increasingly smaller at either end, corresponding with the tapering of the first side band 1401 towards the second side band 1402 at one end, and the tapering of the second side band 1402 towards the first side band 1401 at the other end.

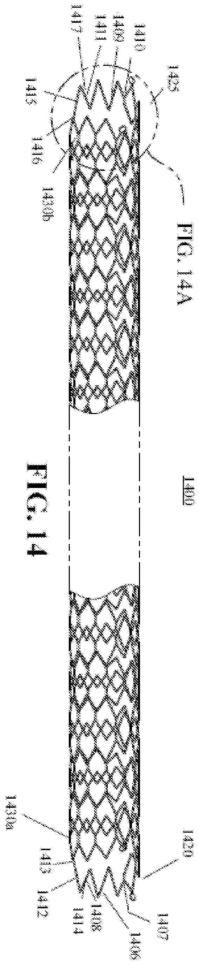

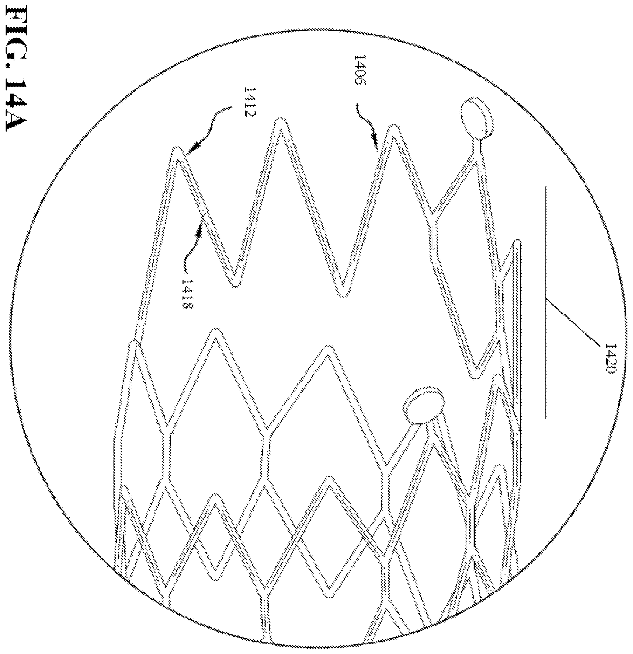

FIG. 14 illustrates the main stent component 1400 in the tubular form, e.g. helically wound. As shown in FIG 14A, which illustrates an enlarged portion of the stent of FIG. 14, the first end band 1406 is connected to the first hook 1412 at connection point 1418, e.g. by welding. Thus, the first end band 1406 and first hook 1412, thereby forming a first cylinder 1420, which is approximately a right cylinder, at one end of the stent. Likewise, the second end band 1409 and second hook 1415 form a second cylinder 1425, which is also approximately a right cylinder, at the other end of the stent.

The end bands may be understood alternatively as continuations of the side bands, such that - for example - first end band 1406 is an extension of the first side band 1401, formed by an undulating pattern extending from the point of connection 1407 where the second side band 1402 tapers to connect with the first side band 1401. Likewise, the second side band 1409 may be understood as an extension of the second side band 1402, formed by an undulating pattern extending from the point of connection 1410 where the first side band 1401 tapers to connect with the second side band 1402.

When the stent of the invention comprises an amorphous metal alloy, it provides the further advantage of enhanced corrosion resistance, resistance to unwanted permanent deformation and higher strength for a given metal thickness. Stents of the present invention comprising amorphous metal alloys exhibit significantly lower conductance or are non-conductive, compared to their crystalline or polycrystalline counterparts. Many medical uses for stents can benefit from such enhanced physical and chemical properties. One embodiment of this invention contemplates intraluminal prosthetic devices comprising at least one amorphous metal alloy combined with components made of other materials, with biocompatible materials being required. This embodiment of the invention may contain one or more amorphous metal alloys. Such alloys provide improved tensile strength, elastic deformation properties, and reduced corrosion potential to the devices.

Amorphous metal alloys, also known as metallic glasses, are disordered metal alloys that do not have long-range crystal structure. Many different amorphous metal alloy compositions are known, including binary, ternary, quaternary, and even quinary alloys. Amorphous metal alloys and their properties have been the subject of numerous reviews (*see, for example,* Amorphous Metal Alloys, edited by F.E. Luborsky, Butterworth & Co, 1983, and references therein). In certain embodiments, the amorphous metal alloys may comprise a metalloid, non-limiting examples of which include silicon, boron, and phosphorus. One possible amorphous metal alloy is an Fe-Cr-B-P alloy. Many other similar alloys are suitable and known to one of ordinary skill in the art.

The stents of the present invention may contain amorphous metal alloys made in a continuous hot extrusion process, as described herein, which possess physical and chemical properties that make them attractive candidates for use in medical devices. For example, amorphous metal alloys may have a tensile strength that is up to ten-fold higher than that of their conventional crystalline or polycrystalline metal counterparts. Also, amorphous metal alloys may have a ten-fold wider elastic range, *i.e.,* range of local strain before permanent deformation occurs. These are important features in medical devices to provide an extended fatigue-resistant lifespan for devices that are subjected to repeated deformations in the body. In addition, these features allow production of smaller or thinner devices that are as strong as their bulkier conventional counterparts.

In other embodiments, the device may contain one or more non-amorphous metals. For example, the device may have components constructed of stainless steel, cobalt chromium ("CoCr"), NiTi or other known materials. With regard to NiTi, the contemplated component may be formed by etching a flat sheet of NiTi into the desired pattern. The flat sheet is formed by rolling the etched sheet into a tubular shape, and optionally welding the edges of the sheet together to form a tubular stent. The details of this method, which has certain advantages, are disclosed in U.S. Patent Nos. 5,836,964 and 5,997,973. Other methods known to those of skill in the art such as laser cutting a tube or etching a tube may also be used to construct a stent of the present invention. A NiTi stent, for example, may be heat treated, as known by those skilled in the art, to take advantage of the shape memory characteristics and/or its super-elasticity.

The amorphous metal alloy or other non-amorphous metal components of this invention may also be combined or assembled with other components, either amorphous metal or otherwise, in order to form intraluminal stents. For example, the amorphous metal alloy or other non-amorphous metal components may be combined with a polymer layer such as a biocompatible polymer, a therapeutic agent (*e.g.,* a healing promoter as described herein) or another metal or metal alloy article (having either a crystalline or amorphous microstructure).

The method of combining or joining the amorphous metal alloy or other non-amorphous metal components to other components can be achieved using methods that are well known in the art. Particularly in the case of non-amorphous metals, the helically coiled main stent component may be secured or otherwise intertwined or joined at the ends to the adjacent helical coils. For example, a biocompatible polymer layer covering all or part of the main stent component may be used to secure the helical coils in its tubular shape for positioning and expansion in the lumen. Other non-limiting examples of securement methods including physical joining (*e.g.,* braiding, weaving, crimping, tying, and press-fitting) and joining by adhesive methods (*e.g.,* gluing, dip coating, and spray coating). Combinations of these methods are also contemplated by this invention.

As a further advantage of the invention, the biocompatible structure may be embedded with drug that will inhibit or decrease cell proliferation or will reduce restenosis. Non-limiting examples of such drugs include for example sirolimus, rapamycin, everolimus and paclitaxol, and analogs of these. In addition, the stent may be treated to have active or passive surface components such as drugs that will be advantageous for a longer time after the stent is embedded in the vessel wall.

Various methods of making amorphous metal alloys are known in the art, examples of which are described further below. While preferred embodiments may be shown and described, various modifications and substitutions may be made without departing from the spirit and scope of the present invention. Accordingly, it is to be understood that the present invention is described herein by way of example, and not by limitation.

### Methods of making amorphous metal alloys

Many different methods may be employed to form amorphous metal alloys. A preferred method of producing medical devices according to the present invention uses a process generally known as heat extrusion, with the typical product being a continuous article such as a wire or a strip. The process does not involve additives commonly used in the bulk process that can render the amorphous metal alloy non-biocompatible and even toxic. Thus, the process can produce highly biocompatible materials. In preferred embodiments, the continuous amorphous metal alloy articles are fabricated by a type of heat extrusion known in the art as chill block melt spinning. Two common chill block melt spinning techniques that produce amorphous metal alloy articles suitable for the medical devices of the present invention are free jet melt-spinning and planar flow casting. In the free jet process, molten alloy is ejected under gas pressure from a nozzle to form a free melt jet that impinges on a substrate surface. In the planar flow method, the melt ejection crucible is held close to a moving substrate surface, which causes the melt to be simultaneously in contact with the nozzle and the moving substrate. This entrained melt flow dampens perturbations of the melt stream and thereby improves ribbon uniformity. (See *e.g.,* Liebermann, H. et al., "Technology of Amorphous Alloys" Chemtech, June 1987). Appropriate substrate surfaces for these techniques include the insides of drums or wheels, the outside of wheels, between twin rollers, and on belts, as is well known in the art.

Suitable planar flow casting and free-jet melt spinning methods for producing amorphous metal alloy components for the medical devices of this invention are described in U.S. Patent Nos. 4,142,571; 4,281,706; 4,489,773, and 5,381,856. For example, the planar flow casting process may comprise the steps of heating an alloy in a reservoir to a temperature 50 - 100 °C above its melting temperature to form a molten alloy, forcing the molten alloy through an orifice by pressurizing the reservoir to a pressure of about 0.5 - 2.0 psig, and impinging the molten alloy onto a chill substrate, wherein the surface of the chill substrate moves past the orifice at a speed of between 300 - 1600 meters/minute and is located between 0.03 to 1 millimeter from the orifice. In embodiments involving free-jet melt spinning, the process may comprise the steps of heating an alloy in a reservoir to a temperature above the melting point of the alloy, ejecting the molten alloy through an orifice in the reservoir to form a melt stream with a velocity between 1-10 meters/second, and impinging the melt stream onto a chill substrate, wherein a surface of the chill substrate moves past the orifice at a speed of between 12 - 50 meters/second.

Besides quenching molten metal (*e.g.,* chill block melt spinning), amorphous metal alloys can be formed by sputter-depositing metals onto a substrate, ion-implantation, and solid-phase reaction. Each of these methods has its advantages and disadvantages. The choice of a particular method of fabrication depends on many variables, such as process compatibility and desired end use of the amorphous metal alloy article.

In some embodiments of the invention, amorphous metal alloy components for stents may be used. These components may be provided in a variety of ways. For example, the component may be produced by machining or processing amorphous metal alloy stock (*e.g.,* a wire, ribbon, rod, tube, disk, and the like). Amorphous metal alloy stock made by chill block melt spinning can be used for such purposes.

It should be understood that the above description is only representative of illustrative examples of embodiments. For the reader's convenience, the above description has focused on a representative sample of possible embodiments, a sample that teaches the principles of the invention. Other embodiments may result from a different combination of portions of different embodiments. The description has not attempted to exhaustively enumerate all possible variations.

## Claims

1. A helical stent (1400) comprising:
a) a first side band (1401) having a first end and a second end, and a second side band (1402) having a first end and a second end connected to the first side band (1401) to form a plurality of cells (1430), wherein the first and second side bands (1401, 1402) each have an undulating pattern, wherein the first end of the first side band (1401) tapers toward the second side band (1402), and the first end of the second side band (1402) tapers toward the first side band (1401);
b) a first end band (1406) comprising a series of struts forming a continuation of the first side band (1401), said first end band (1406) having a first end (1407) and a second end (1408), wherein the first end (1407) of said first end band is (1406) connected to the second end of the first side band (1401), and a second end band (1409) comprising a series of struts forming a continuation of the second side band (1402), said second end band (1409) having a first end (1410) and a second end (1411), wherein the first end (1410) of the second end band (1409) is connected to the second end of the second side band (1402), and
**characterized in that**
the second end (1408) of said first end band (1406) is connected to a first hook (1412) extending from the second side band (1402) and
the second end (1411) of said second end band (1409) is connected to a second hook (1415) extending from the first side band (1401).

2. The stent according to claim 1, wherein the first hook (1412) and the first end band (1406) form a first cylinder, wherein the first cylinder is approximately a right cylinder.

3. The stent according to claims 1 or 2, wherein the first hook (1412) is connected to the second end (1408) of the first end band (1406) by welding.

4. The stent according to any one of claims 1 to 3, wherein the second hook (1415) and the second end band (1409) form a second cylinder, wherein the second cylinder is approximately a right cylinder.

5. The stent according to any one of claims 1 to 4, wherein the second hook (1415) is connected to the second end (1411) of the second end band (1409) by welding.

6. The stent according to any one of claims 1 to 5, wherein the first side band (1401) and second side band (1402) are connected by cross struts (1403).

7. The stent according to any one of claims 1 to 6, wherein at least one of said cells (1430) has a different size from at least one other of said cells.

8. The stent according to any one of claims 1 to 7, wherein the size of the cells (1430) get smaller near either end of the stent (1400).

9. A helical stent (1400) according to any of claims 1 to 8 and a polymer material (5),
wherein said first hook (1412) is arranged to align with the second end (1408) of the first end band (1406) and said second hook (1415) is arranged to align with the second end (1411) of the second end band (1409).

10. The stent according to claim 9, wherein the polymer material is a fiber mesh.

11. The stent according to claim 9, wherein the polymer material is a porous material.

12. The stent according to claim 9, wherein the polymer material is a durable material.

13. The stent according to any one of claims 9 to 12, wherein the polymer material extends across the entire length of the stent (1400).

14. The stent according to any one of claims 9 to 13, wherein the polymer material covers an end of the stent (1400).

15. The stent according to any one of claims 9 to 14, wherein the polymer material has fenestrations.

16. The stent according to any one of claims 9 to 10, wherein the polymer material is ePTFE.

17. The stent according to any one of claims 9 to 16, wherein the polymer material provides longitudinal rigidity to the stent.

18. The stent according to any one of claims 9 to 17, wherein the polymer material provides structural support for the stent.

19. A method of making a helical stent according to claim 9, said method comprising the steps of:
a) aligning the second end of the first end band with the first hook of the helical stent;
b) connecting said second end of the first end band with the first hook;
c) aligning the second end of the second end band with the second hook;
d) connecting said second end of the second end band with the second hook; and
e) applying said polymer material.

20. The method according to claim 19, further comprising the step of welding the first hook to the second end of the first end band.

21. The method according to claim 19, further comprising the step of welding the second hook to the second end of the second end band.

22. The method according to claim 19, where the step of applying said polymer material comprises electrospinning.

## Patentansprüche

1. Spiralförmiger Stent (1400), aufweisend:
a) ein erstes Seitenband (1401), das ein erstes Ende und ein zweites Ende aufweist, und ein zweites Seitenband (1402), das ein erstes Ende und ein zweites Ende aufweist und mit dem ersten Seitenband (1401) verbunden ist, um eine Mehrzahl an Zellen (1430) auszubilden, wobei das erste und das zweite Seitenband (1401, 1402) jeweils ein wellenförmiges Muster aufweisen, wobei das erste Ende des ersten Seitenbandes (1401) in Richtung des zweiten Seitenbandes (1402) spitz zuläuft und das erste Ende des zweiten Seitenbandes (1402) in Richtung des ersten Seitenbandes (1401) spitz zuläuft;
b) ein erstes Endband (1406), das eine Reihe an Streben aufweist, die eine Fortsetzung des ersten Seitenbandes (1401) bilden, wobei das erste Endband (1406) ein erstes Ende (1407) und ein zweites Ende (1408) aufweist, wobei das erste Ende (1407) des ersten Endbandes (1406) mit dem zweiten Ende des ersten Seitenbandes (1401) verbunden ist, und ein zweites Endband (1409), das eine Reihe an Streben aufweist, die eine Fortsetzung des zweiten Seitenbandes (1402) bilden, wobei das zweite Endband (1409) ein erstes Ende (1410) und ein zweites Ende (1411) aufweist, wobei das erste Ende (1410) des zweiten Endbandes (1409) mit dem zweiten Ende des zweiten Seitenbandes (1402) verbunden ist, und
**dadurch gekennzeichnet, dass**
das zweite Ende (1408) des ersten Endbandes (1406) mit einem ersten Haken (1412) verbunden ist, der von dem zweiten Seitenband (1402) aus verläuft, und
das zweite Ende (1411) des zweiten Endbandes (1409) mit einem zweiten Haken (1415) verbunden ist, der von dem ersten Seitenband (1401) aus verläuft.

2. Stent nach Anspruch 1, wobei der erste Haken (1412) und das erste Endband (1406) einen ersten Zylinder bilden, wobei der erste Zylinder ein annähernd gerader Zylinder ist.

3. Stent nach Anspruch 1 oder 2, wobei der erste Haken (1412) mit dem zweiten Ende (1408) des ersten Endbandes (1406) durch Schweißen verbunden ist.

4. Stent nach einem der Ansprüche 1 bis 3, wobei der zweite Haken (1415) und das zweite Endband (1409) einen zweiten Zylinder bilden, wobei der zweite Zylinder ein annähernd gerader Zylinder ist.

5. Stent nach einem der Ansprüche 1 bis 4, wobei der zweite Haken (1415) mit dem zweiten Ende (1411) des zweiten Endbandes (1409) durch Schweißen verbunden ist.

6. Stent nach einem der Ansprüche 1 bis 5, wobei das erste Seitenband (1401) und das zweite Seitenband (1402) durch Querstreben (1403) verbunden sind.

7. Stent nach einem der Ansprüche 1 bis 6, wobei zumindest eine der Zellen (1430) eine Größe aufweist, die sich zumindest von einer anderen der Zellen unterscheidet.

8. Stent nach einem der Ansprüche 1 bis 7, wobei die Größe der Zellen (1430) in der Nähe jedes Endes des Stents (1400) kleiner wird.

9. Spiralförmiger Stent (1400) nach einem der Ansprüche 1 bis 8 und ein Polymermaterial (5),
wobei der erste Haken (1412) so angeordnet ist, dass er sich auf das zweite Ende (1408) des ersten Endbandes (1406) ausrichtet und der zweite Haken (1415) so angeordnet ist, dass er sich auf das zweite Ende (1411) des zweiten Endbandes (1409) ausrichtet.

10. Stent nach Anspruch 9, wobei das Polymermaterial ein Fasernetz ist.

11. Stent nach Anspruch 9, wobei das Polymermaterial ein poröses Material ist.

12. Stent nach Anspruch 9, wobei das Polymermaterial ein dauerhaftes Material ist.

13. Stent nach einem der Ansprüche 9 bis 12, wobei das Polymermaterial über die gesamte Länge des Stents (1400) verläuft.

14. Stent nach einem der Ansprüche 9 bis 13, wobei das Polymermaterial ein Ende des Stents (1400) bedeckt.

15. Stent nach einem der Ansprüche 9 bis 14, wobei das Polymermaterial Fenestrationen aufweist.

16. Stent nach einem der Ansprüche 9 bis 10, wobei das Polymermaterial ePTFE ist.

17. Stent nach einem der Ansprüche 9 bis 16, wobei das Polymermaterial dem Stent eine Längssteifigkeit verleiht.

18. Stent nach einem der Ansprüche 9 bis 17, wobei das Polymermaterial dem Stent strukturellen Halt verleiht.

19. Verfahren zur Herstellung eines spiralförmigen Stents nach Anspruch 9, wobei das Verfahren die folgenden Schritte aufweist:
a) Ausrichten des zweiten Endes des ersten Endbandes auf den ersten Haken des spiralförmigen Stents;
b) Verbinden des zweiten Endes des ersten Endbandes mit dem ersten Haken;
c) Ausrichten des zweiten Endes des zweiten Endbandes auf den zweiten Haken;
d) Verbinden des zweiten Endes des zweiten Endbandes mit dem zweiten Haken; und
e) Aufbringen des Polymermaterials.

20. Verfahren nach Anspruch 19, ferner aufweisend den Schritt des Schweißens des ersten Hakens an das zweite Ende des ersten Endbandes.

21. Verfahren nach Anspruch 19, ferner aufweisend den Schritt des Schweißens des zweiten Hakens an das zweite Ende des zweiten Endbandes.

22. Verfahren nach Anspruch 19, wobei der Schritt des Aufbringens des Polymermaterials ein Elektrospinnen aufweist.

## Revendications

1. Endoprothèse hélicoïdale (1400) comprenant :
a) une première bande latérale (1401) ayant une première extrémité et une seconde extrémité, et une seconde bande latérale (1402) ayant une première extrémité et une seconde extrémité raccordées à la première bande latérale (1401) pour former une pluralité de cellules (1430), dans laquelle les première et seconde bandes latérales (1401, 1402) ont chacune un motif ondulé, dans laquelle la première extrémité de la première bande latérale (1401) rétrécit en direction de la seconde bande latérale (1402), et la première extrémité de la seconde bande latérale (1402) rétrécit en direction de la première bande latérale (1401) ;
b) une première bande d'extrémité (1406) comprenant une série d'entretoises formant une continuation de la première bande latérale (1401), ladite première bande d'extrémité (1406) ayant une première extrémité (1407) et une seconde extrémité (1408), dans laquelle la première extrémité (1407) de ladite première bande d'extrémité (1406) est raccordée à la seconde extrémité de la première bande latérale (1401), et une seconde bande d'extrémité (1409) comprenant une série d'entretoises formant une continuation de la seconde bande latérale (1402), ladite seconde bande latérale (1409) ayant une première extrémité (1410) et une seconde extrémité (1411), dans laquelle la première extrémité (1410) de la seconde bande d'extrémité (1409) est raccordée à la second extrémité de la seconde bande latérale (1402), et
**caractérisée en ce que**
la seconde extrémité (1408) de ladite première bande d'extrémité (1406) est raccordée à un premier crochet (1412) s'étendant à partir de la seconde bande latérale (1402) et
la seconde extrémité (1411) de ladite seconde bande d'extrémité (1409) est raccordée à un second crochet (1415) s'étendant à partir de la première bande latérale (1401).

2. Endoprothèse selon la revendication 1, dans laquelle le premier crochet (1412) et la première bande d'extrémité (1406) forment un premier cylindre, dans laquelle le premier cylindre est approximativement un cylindre droit.

3. Endoprothèse selon les revendications 1 ou 2, dans laquelle le premier crochet (1412) est raccordé à la seconde extrémité (1408) de la première bande d'extrémité (1406) par soudure.

4. Endoprothèse selon l'une quelconque des revendications 1 à 3, dans laquelle le second crochet (1415) et la seconde bande d'extrémité (1409) forment un second cylindre, dans laquelle le second cylindre est approximativement un cylindre droit.

5. Endoprothèse selon l'une quelconque des revendications 1 à 4, dans laquelle le second crochet (1415) est raccordé à la seconde extrémité (1411) de la seconde bande d'extrémité (1409) par soudure.

6. Endoprothèse selon l'une quelconque des revendications 1 à 5, dans laquelle la première bande latérale (1401) et la seconde bande latérale (1402) sont raccordées par des entretoises croisées (1403).

7. Endoprothèse selon l'une quelconque des revendications 1 à 6, dans laquelle au moins l'une desdites cellules (1430) a une taille différente d'au moins une autre desdites cellules.

8. Endoprothèse selon l'une quelconque des revendications 1 à 7, dans laquelle la taille des cellules (1430) diminue à proximité de l'une ou l'autre extrémité de l'endoprothèse (1400).

9. Endoprothèse hélicoïdale (1400) selon l'une quelconque des revendications 1 à 8 et un matériau polymère (5),
dans laquelle ledit premier crochet (1412) est agencé pour s'aligner sur la seconde extrémité (1408) de la première bande d'extrémité (1406) et ledit second crochet (1415) est agencé pour s'aligner sur la seconde extrémité (1411) de la seconde bande d'extrémité (1409).

10. Endoprothèse selon la revendication 9, dans laquelle le matériau polymère est un treillis de fibres.

11. Endoprothèse selon la revendication 9, dans laquelle le matériau polymère est un matériau poreux.

12. Endoprothèse selon la revendication 9, dans laquelle le matériau polymère est un matériau durable.

13. Endoprothèse selon l'une quelconque des revendications 9 à 12, dans laquelle le matériau polymère s'étend sur la longueur totale de l'endoprothèse (1400).

14. Endoprothèse selon l'une quelconque des revendications 9 à 13, dans laquelle le matériau polymère couvre une extrémité de l'endoprothèse (1400).

15. Endoprothèse selon l'une quelconque des revendications 9 à 14, dans laquelle le matériau polymère comprend des fenestrations.

16. Endoprothèse selon l'une quelconque des revendications 9 à 10, dans laquelle le matériau polymère est l'ePTFE.

17. Endoprothèse selon l'une quelconque des revendications 9 à 16, dans laquelle le matériau polymère fournit une rigidité longitudinale à l'endoprothèse.

18. Endoprothèse selon l'une quelconque des revendications 9 à 17, dans laquelle le matériau polymère fournit le support structural pour l'endoprothèse.

19. Procédé de fabrication d'une endoprothèse hélicoïdale selon la revendication 9, ledit procédé comprenant les étapes de :
a) alignement de la seconde extrémité de la première bande d'extrémité sur le premier crochet de l'endoprothèse hélicoïdale ;
b) raccordement de ladite seconde extrémité de la première bande d'extrémité avec le premier crochet ;
c) alignement de la seconde extrémité de la seconde bande d'extrémité avec le second crochet ;
d) raccordement de ladite seconde extrémité de la seconde bande d'extrémité avec le second crochet ; et
e) application dudit matériau polymère.

20. Procédé selon la revendication 19, comprenant en outre l'étape de soudure du premier crochet à la seconde extrémité de la première bande d'extrémité.

21. Procédé selon la revendication 19, comprenant en outre l'étape de soudure du second crochet à la seconde extrémité de la seconde bande d'extrémité.

22. Procédé selon la revendication 19, dans lequel l'étape d'application dudit matériau polymère comprend l'électrofilature.
